(19) Europäisches Patentamt
European Patent Office
Office européen des brevets

(11) Veröffentlichungsnummer : **0 032 374 B1**

(12) **EUROPÄISCHE PATENTSCHRIFT**

(45) Veröffentlichungstag der Patentschrift : **09.03.83**

(51) Int. Cl.³ : **C 07 C 59/52**, C 07 C 51/377

(21) Anmeldenummer : **81100047.0**

(22) Anmeldetag : **07.01.81**

(54) **Verfahren zur Herstellung von p-Hydroxyphenylessigsäure.**

(30) Priorität : **17.01.80 DE 3001511**

(43) Veröffentlichungstag der Anmeldung : **22.07.81 Patentblatt 81/29**

(45) Bekanntmachung des Hinweises auf die Patenterteilung : **09.03.83 Patentblatt 83/10**

(84) Benannte Vertragsstaaten : **AT BE CH DE FR GB IT LI NL**

(56) Entgegenhaltungen :
**EP A 0 003 825**
**DE A 2 820 854**

**CHEMISCHES ZENTRALBLATT, Band 112, Nr. 17, 23 April 1941, E. SPÄTH et al. « Über die Inhaltsstoffe des roten Sandelholzes. III. Mitt. Die Synthese des Pterostilbens », Seiten 2256 bis 2257.**

**HOUBEN WEYL « Methoden der organischen Chemie », 4. Auflage, Band VI/1c, Teil 2 1976, GEORG THIEME VERLAG, Stuttgart, Seite 1058.**

(73) Patentinhaber : **HOECHST AKTIENGESELLSCHAFT**
**Postfach 80 03 20**
**D-6230 Frankfurt/Main 80 (DE)**

(72) Erfinder : **Spielmann, Werner, Dr.**
**Johann-Strauss-Strasse 18a**
**D-6233 Kelkheim (Taunus) (DE)**
Erfinder : **Schaeffer, Georg, Dr.**
**Herderstrasse 58**
**D-6238 Hofheim am Taunus (DE)**

**EP 0 032 374 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von p-Hydroxyphenylessigsäure

p-Hydroxyphenylessigsäure ist ein wertvolles Zwischenprodukt auf verschiedenen Sachgebieten, insbesondere auf dem Pharmasektor, z. B. zur Herstellung antibiotisch wirksamer Substanzen (US-PS 2 487 018), von β-Blockern (GB-PS 1 285 038), etc.

Zur p-Hydroxyphenylessigsäure kann man auf einer Reihe bekannter Wege gelangen. Ein neuerer Weg (EP-OS 3 825) geht aus von o-Chlorphenol und Glyoxylsäure und kommt in drei Stufen schließlich zur p-Hydroxyphenylessigsäure. Die drei Stufen dieses Verfahrens sind :

a) Umsetzung von o-Chlorphenol mit Glyoxylsäure zu 3-Chlor-4-hydroxymandelsäure,

b) Reduktion der 3-Chlor-4-hydroxymandelsäure zu 3-Chlor-4-hydroxyphenylessigsäure — vorzugsweise mittels Jodwasserstoffsäure HJ in Gegenwart von rotem Phosphor, und

c) Abspaltung des Chlors aus der 3-Chlor-4-hydroxyphenylessigsäure — vorzugsweise durch katalytische Hydrierung an einem Pd-Katalysator.

Der Umweg über die 3-Chlor-4-hydroxymandelsäure wird hier deswegen beschritten, weil sich die (nicht weiter substituierte) p-Hydroxymandelsäure « nicht auf einfache Weise » zur p-Hydroxyphenylessigsäure reduzieren lassen soll. Mit Reduktion « auf einfache Weise » ist nach der EP-OS insbesondere die für das Verfahren dieser EP-OS bevorzugte Reduktion mit Jodwasserstoffsäure HJ — die im übrigen auch in die Lehrbücher der organischen Chemie aufgenommene Standardmethode für die Reduktion der (unsubstituierten) Mandelsäure zur (unsubstituierten) Phenylessigsäure [siehe z. B. Fieser & Fieser, Lehrbuch der organischen Chemie, Verlag Chemie GmbH, Weinheim/Bergstraße, S. 784/85 (1960)] — gemeint.

Nach der (prioritätsjüngeren) DE-OS 28 20 854 ist jedoch offensichtlich auch die direkte Reduktion der p-Hydroxymandelsäure zu der gewünschten p-Hydroxyphenylessigsäure auf verschiedenen « einfachen » Wegen gelungen. Nach dieser DE-OS erfolgt die Reduktion der p-Hydroxymandelsäure zur p-Hydroxyphenylessigsäure

a) durch katalytische Hydrierung an einem Pd-Katalysator in mineralsauer — insbesondere salzsaurer — Lösung,

b) mit unterphosphoriger Säure $H_3PO_2$ oder einem Salz derselben, oder

c) mit einem Chrom-(II)-Salz, das in situ durch Reduktion eines Chrom-(III)-Salzes mit Zn/Säure hergestellt wird.

In dem nach der Methode a) (katalytische Hydrierung) arbeitenden Beispiel (Nr. 1) der DE-OS wird eine Ausbeute an p-Hydroxyphenylessigsäure von 85 % d. Th. angegeben ; für diese Methode ist allerdings — wie eigene Versuche gezeigt haben — nachteilig, daß die p-Hydroxyphenylessigsäure hier in ziemlich stark (rot) verfärbtem Zustand mit einem Fp. von nur 145-147 °C (theoretischer Fp. 150 °C !) anfällt. Außerdem ist wegen der Notwendigkeit der Neutralisation der verwendeten Mineralsäure der dabei auftretende Salzanfall nicht zu vernachlässigen.

Bei Methode b) (Reduktion mit $H_3PO_2$ oder deren Salzen) ist nach dem diesbezüglichen Beispiel (Nr. 3) die Ausbeute an p-Hydroxyphenylessigsäure von 67 % d. Th. nicht ganz zufriedenstellend, und für Methode c) [Reduktion mit Chrom-(II)-Salz bzw. Chrom-(III)-Salz/Zn/Säure] ist trotz der hohen Ausbeute an p-Hydroxyphenylessigsäure von 97 % d. Th. (Beispiel 2) der beträchtliche Bedarf und Anfall von Metallsalzen und die damit verbundene Abwasserbelastung von Nachteil.

Von der Reduktion der p-Hydroxymandelsäure zur p-Hydroxyphenylessigsäure mit Jodwasserstoffsäure HJ etwa analog der Reduktion der (unsubstituierten) Mandelsäure zur (unsubstituierten) Phenylessigsäure ist in der genannten DE-OS jedoch nirgends die Rede. Es war also nach wie vor davon auszugehen, daß gerade die Methode der Reduktion mittels HJ nicht auf die p-Hydroxymandelsäure anwendbar ist. Bezüglich dieser Methode wurde somit dem durch die EP-OS 3 825 konstituierten Vorurteil durch die DE-OS 28 20 854 keinerlei Abbruch getan ; die DE-OS kann sogar — weil sie von den verschiedenen möglichen direkten Reduktionsmethoden so relativ wenig günstige wie die Cr(II)-Salz-Methode, nicht aber die an sich günstigere HJ-Methode benutzt — eher als eine Bestätigung des genannten Vorurteils aufgefaßt werden.

Da man somit nach dem Stand der Technik davon ausgehen mußte, daß die Reduktion der p-Hydroxymandelsäure zur p-Hydroxyphenylessigsäure mit Jodwasserstoffsäure HJ nicht auf einfache Weise möglich ist, war weiter auch anzunehmen, daß etwa die aus der heterocyclischen Reihe bekannte « Eintopfreaktion » zur Herstellung von Pyrrylessigsäure aus Pyrrol oder Pyrrolderivaten, Glyoxylsäure und HJ, bei der wohl entsprechende Pyrrylhydroxyessigsäuren als Zwischenstufen auftreten, auf die Herstellung der p-Hydroxyphenylessigsäure nicht übertragbar ist. Die erwähnte « Eintopfreaktion » in der Pyrrolreihe ist beschrieben in Canad. J. Chem. 48 (1970), S. 139-143 und kann durch folgende Summengleichung wiedergegeben werden :

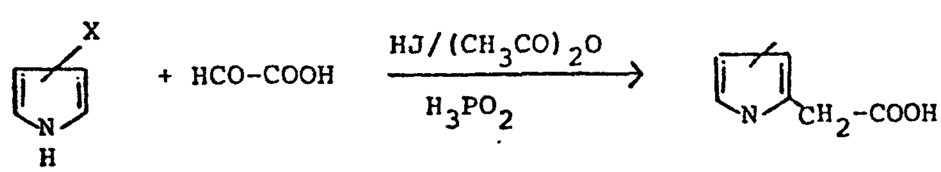


Pyrrol-(Derivat)

Pyrrylessigsäure

In dem Bestreben, die Verfahren des Standes der Technik zur Herstellung der p-Hydroxyphenylessigsäure weiter zu verbessern, wurde bereits vorgeschlagen (DE-A1 29 44 480.2, EP-A10 028 375) die in der DE-OS 28 20 854 (sowie etwa deren BE-Äquivalent, der BE-PS 867 289) beschriebene Methode der katalytischen Hydrierung der p-Hydroxymandelsäure dadurch wirtschaftlicher zu gestalten, daß man diese katalytische Hydrierung in einer wässrigen Lösung frei von Mineralsäure, insbesondere frei von Salzsäure, durchführt. Bei gegenüber der Arbeitsweise gemäß DE-OS 28 20 854 praktisch gleicher Ausbeute (etwa 85 %) entfallen hier die mit der Verwendung von Mineralsäure verbundenen Korrosionsetc. Probleme.

In Fortführung dieser Arbeiten wurde nun gefunden, daß sich p-Hydroxymandelsäure auch mit Jodwasserstoffsäure HJ in Gegenwart von rotem Phosphor in ausgezeichneter Weise zu p-Hydroxyphenylessigsäure reduzieren läßt.

Das Gelingen dieser Reduktion (mit quantitativem Umsatz der p-Hydroxymandelsäure zu p-Hydroxyphenylessigsäure und mit Ausbeuten an isoliertem Produkt bis zu etwa 95 % d. Th. !) war insbesondere im Hinblick auf das durch die EP-OS 3 825 konstituierte und durch die DE-PS 28 20 854 praktisch bestätigte Vorurteil gegen die Möglichkeit der direkten Reduktion der p-Hydroxymandelsäure zur p-Hydroxyphenylessigsäure mit HJ überraschend.

Nachdem dieses Vorurteil nun überwunden war, konnte auch die in der heterocyclischen Reihe aus Canad. J. Chem. 48 (1970) S. 139-143 bekannte « Eintopfreaktion » auf die Herstellung der p-Hydroxyphenylessigsäure übertragen werden.

Erfindungsgegenstand ist somit ein Verfahren zur Herstellung von p-Hydroxyphenylessigsäure durch Reduktion der p-Hydroxymandelsäure, das dadurch gekennzeichnet ist, daß man die Reduktion mit HJ in Gegenwart von rotem Phosphor durchführt.

Die p-Hydroxymandelsäure kann für dieses Verfahren als solche oder als Hydrat oder auch in Form ihrer Salze — vorzugsweise der Alkalisalze, und insbesondere des Na-Salzes — oder von deren Hydraten eingesetzt werden.

Ansonsten wird die Reduktion mit HJ/rotem Phosphor in der für derartige Reduktionen üblichen Weise, wie z. B. von der Überführung von unsubstituierter Mandelsäure in unsubstituierte Phenylessigsäure bekannt, durchgeführt; selbstverständlich kann die Jodwasserstoffsäure auch erst im Reduktionsansatz aus einem ihrer Salze (z. B. KJ) und einer Säure (wie z. B. Phosphorsäure) erzeugt werden. Eine zweckmäßige Art der Reaktionsdurchführung besteht beispielsweise darin, daß man p-Hydroxymandelsäure in wässriger Jodwasserstoffsäure in Gegenwart von rotem Phosphor kurzzeitig auf Rückflußtemperatur erhitzt und die Jodwasserstoffsäure anschließend durch Destillation wieder zurückgewinnt ; die Zurückgewinnung der Jodwasserstoffsäure gelingt hier durchweg nahezu quantitativ.

Auch wenn anstelle der freien p-Hydroxymandelsäure eines ihrer Salze wie z. B. das Na-Salz eingesetzt wird, läßt sich die freie Jodwasserstoffsäure praktisch quantitativ zurückgewinnen.

Der heiße Destillationsrückstand wird anschließend mit Wasser verdünnt, unumgesetzter Phosphor heiß abfiltriert und das Filtrat auf Raumtemperatur kalt gerührt, wobei die p-Hydroxyphenylessigsäure in besonders reiner Form auskristallisiert. Weiteres Produkt läßt sich noch durch Einengen der Mutterlauge gewinnen, so daß man hierbei zu einer Gesamtausbeute von etwa 95 % d. Th. kommt.

In einer bevorzugten Ausführungsform der Erfindung wird die als Ausgangsprodukt für die Reduktion verwendete p-Hydroxymandelsäure in situ in an sich bekannter Weise [s. z. B. Houben-Weyl, Methoden der Organischen Chemie, Bd. VI/1c (1976), S. 1057-58] aus Phenol und Glyoxylsäure hergestellt. Wenn man bei dieser Ausführungsart Phenol und Glyoxylsäure mit Jodwasserstoffsäure in Gegenwart von rotem Phosphor vereinigt, gelangt man in einer « Eintopfreaktion » ohne Isolierung der als Zwischenstufe auftretenden p-Hydroxymandelsäure direkt zur p-Hydroxyphenylessigsäure. Für diese Umsetzung ist es notwendig, Phenol und Glyoxylsäure etwa im Molverhältnis 1 : 1 zu verwenden ; die Verwendung eines Phenol-Überschusses, zweckmäßig von etwa 1,3 Mol/Mol Glyoxylsäure, ist jedoch zwecks Zurückdrängung der Bildung von 2,4-Dicarboxymethylphenol vorteilhaft.

Als Lösungsmittel dient auch hier wie bei der normalen Ausführungsart des erfindungsgemäßen Verfahrens Jodwasserstoffsäure, welche destillativ zurückgewonnen werden kann.

Phenol und Glyoxylsäure werden hier glatt und mit nahezu quantitativem Umsatz der Glyoxylsäure zu einem Isomerengemisch von etwa 85 % p- und etwa 15 % o-Hydroxyphenylessigsäure umgesetzt. Daraus läßt sich durch Kristallisation analog zu der Aufarbeitungsmethode bei der normalen Durchführungsart des Verfahrens reine p-Hydroxyphenylessigsäure in bis zu etwa 53 %iger Ausbeute, bezogen auf die eingesetzte Glyoxylsäure gewinnen. Die Ausbeute liegt damit etwa in der Größenordnung des 2-stufigen Reaktionswegs mit Isolierung der p-Hydroxymandelsäure. Die wegen der guten Wasserlöslichkeit schwierige Isolierung der p-Hydroxymandelsäure bzw. von deren Salzen wird durch die erfindungsgemäße « Eintopf »-Variante vermieden.

Die sowohl für die normale als auch für die bevorzugte Ausführungsform der Erfindung notwendige Jodwasserstoffsäure kann im Prinzip in allen möglichen, gegen die Reaktionsausgangs- und endstoffe inerten Lösungsmitteln wie z. B. in Wasser oder Essigsäure etc. eingesetzt werden. Bevorzugt ist die Verwendung von wässriger — insbesondere von (bei Normaldruck) konstant siedender etwa 57 (gewichts-)%iger — Jodwasserstoffsäure.

Die für die bevorzugte Ausführungsform des Verfahrens nötige Glyoxylsäure wird zweckmäßig

ebenfalls in Form einer wässrigen Lösung, insbesondere der handeslüblichen etwa 50 (gew.-)%igen Lösung, eingesetzt. Das mit der Glyoxylsäure eingebrachte Wasser läßt sich bei der destillativen Rückgewinnung der Jodwasserstoffsäure als Vorlauf leicht abtrennen. Führt man die Reaktion in sonst gleicher Weise etwa mit 80 (Gew.-)% wässriger Glyoxylsäure (= Glyoxylsäure-Monohydrat) durch, kann die Jodwasserstoffsäure direkt und ohne Abtrennung eines Wasservorlaufs zurückgewonnen werden.

Als Reaktionstemperaturen kommen Temperaturen zwischen Raumtemperatur (etwa 20 °C) und etwa 150 °C in Frage ; bevorzugt sind Temperaturen zwischen etwa Raumtemperatur und 100 °C.

Die normale Ausführungsart des erfindungsgemäßen Verfahrens stellt wegen der dabei erzielten hohen Ausbeuten (bis etwa 95 % d. Th.) und der hohen Produktreinheit gegenüber der wohl nächstvergleichbaren katalytischen Hydrierung gemäß DE-OS 28 20 854 (Ausbeute 85 %) sowie der DE-A1 29 44 480.2 und der EP-A1 00 283 75 (Ausbeute ebenfalls etwa 85 %) auch in Abetracht der Notwendigkeit des Einsatzes von Jodwasserstoffsäure ein fortschrittliches oder zumindest ebenbürtiges Verfahren dar, weil die Jodwasserstoffsäure immer wieder ohne besonderen Aufwand zurückgewonnen werden kann. Die mit etwas höherer Ausbeute (97 %) arbeitende Chrom-(II)-Salzmethode gemäß DE-OS 28 20 854 ist wegen der dabei auftretenden nicht unbeträchtlichen Abwasserprobleme insgesamt sicher weniger vorteilhaft.

Der Fortschritt gegenüber dem Verfahren der EP-OS 3 825 besteht in der Vermeidung des Umwegs über die 3-Chlor-4-hydroxymandelsäure.

Der Fortschritt der « Eintopf »-Ausführungsform der Erfindung ist in erster Linie durch die Einfachheit der Durchführung begründet.

Die folgenden Beispiele sollen nun der näheren Erläuterung der Erfindung dienen.

Beispiel 1

10 kg p-Hydroxymandelsäure-Hydrat (= 53 Mol), 34 kg = 20 l 57 %ige Jodwasserstoffsäure und 1,15 kg (= 37 Mol) roter Phosphor werden 1 Stunde unter Rückfluß erhitzt und anschließend die Jodwasserstoffsäure bis 140 °C Sumpftemperatur abdestilliert. Weitere Jodwasserstoffsäure kann noch durch Anlegen eines leichten Vakuum von 19 999 Pa (150 Torr) aus dem Destillationsrückstand gewonnen werden. Insgesamt erhält man 19,5 l Jodwasserstoffsäure zum Wiedereinsatz zurück.

Der Destillationsrückstand wird mit 15 l Wasser verdünnt und heiß abgesaugt, wobei man 0,37 kg roten Phosphor zurückgewinnt.

Man läßt das Filtrat über Nacht auf Raumtemperatur kaltrühren, saugt die ausgefallene p-Hydroxyphenylessigsäure ab und wäscht mit wenig Wasser jodfrei. Nach Trocknen im Vakuum bei 80 °C erhält man 6,5 kg (= 80,6 % d. Th.) Produkt. Weitere p-Hydroxyphenylessigsäure erhält man durch Einengen von Mutterlauge und Waschwasser und Kristallisation wie oben.

Gesamtausbeute : 7,76 kg = 95 % d. Th., Fp. 150 °C

Beispiel 2

75 kg p-Hydroxymandelsäure-Na -Salz-Hydrat (= 360 Mol), 260 kg = 57 %ige Jodwasserstoffsäure und 7,5 kg roter Phosphor (= 242 Mol) werden 1 Stunde unter Rückfluß erhitzt. Dann destilliert man über eine Füllkörperkolonne bei einem Rücklaufverhältnis von 2 : 1 einen Vorlauf von ca. 28 l Wasser ab. Anschließend werden wie in Beispiel 1 168 kg wiedereinsetzbare Jodwasserstoffsäure abdestilliert.

Man verdünnt den Destillationsrückstand mit 155 l Wasser und arbeitet wie in Beispiel 1 auf.

Ausbeute : 52 kg p-Hydroxyphenylessigsäure (= 95 % d. Th.), Fp. 150 °C

Beispiel 3

Zu einer gut gerührten Mischung aus 1,22 kg Phenol (= 13 Mol), 190 g rotem Phosphor (6,1 Mol) und 4 l = 6,8 kg 57 %ige Jodwasserstoffsäure tropft man innerhalb 2 Stunden bei 25 °C 1,48 kg 50 %ige wässrige Glyoxylsäure (= 10 Mol) und rührt über Nacht bei 25 °C nach.

Anschließend heizt man für 1 Stunde auf 90 °C und destilliert bei 19 999 Pa (150 Torr) über einer Füllkörperkolonne bei einem Rücklaufverhältnis von 2 : 1 einen Vorlauf von ca. 650 g eine Phenol/Wasser-Azeotropes ab.

Anschließend gehen 6,8 kg phenolhaltige Jodwasserstoffsäure über, die bei folgenden Ansätzen wieder eingesetzt wird. Der heiße Destillationsrückstand wird mit 3 l Wasser verdünnt, nicht umgesetzter roter Phosphor abgesaugt und das Filtrat auf 5 °C kaltgerührt. Man saugt die ausgefallene p-Hydroxyphenylessigsäure ab, wäscht 3 mal mit je 400 ml Wasser jodfrei und trocknet bei 80 °C im Vakuum. Ausbeute : 741 g = 48,8 % d. Th.

Verwendet man das Waschwasser der p-Hydroxyphenylessigsäure bei folgenden Ansätzen zum Verdünnen des Destillationsrückstandes, erhöht sich die Ausbeute auf : 811 g p-Hydroxyphenylessigsäure (= 53,4 % d. Th.), Fp. 150 °C.

**Ansprüche**

1. Verfahren zur Herstellung von p-Hydroxyphenylessigsäure durch Reduktion der p-Hydroxymandelsäure, dadurch gekennzeichnet, daß man die Reduktion mit HJ in Gegenwart von rotem Phosphor durchführt.

2. Verfahren nach Anspruch 1, dadurch gekennzeichnet, daß man die als Ausgangsprodukt für die Reduktion verwendete p-Hydroxymandelsäure in situ aus Phenol und Glyoxylsäure herstellt.

3. Verfahren nach den Ansprüchen 1 bis 2, dadurch gekennzeichnet, daß man den Jodwasserstoff HJ in Form einer wässrigen Lösung, vorzugsweise in Form der konstant siedenden etwa 57 (Gew.-)%igen wässrigen Jodwasserstoffsäure, einsetzt.

4. Verfahren nach Anspruch 2, dadurch gekennzeichnet, daß man die Glyoxylsäure in Form der handelsüblichen etwa 50 (gew.-)%igen wässrigen Lösung einsetzt.

**Claims**

1. Process for the manufacture of p-hydroxyphenylacetic acid by reduction of p-hydroxymandelic acid, characterized in carrying out the reduction with HI in the presence of red phosphorus.

2. The process as claimed in claim 1, characterized in preparing the p-hydroxymandelic acid used as starting material for the reduction in situ from phenol and glyoxylic acid.

3. The process as claimed in claim 1 to 2, characterized in using the hydrogen iodide HI in the form of an aqueous solution, preferably an about 57 % by weight aqueous hydroiodic acid having a constant boiling point.

4. Process as claimed in claim 2, characterized in using the glyoxylic acid in the form of a commercial, about 50 % by weight aqueous solution.

**Revendications**

1. Procédé pour préparer l'acide p-hydroxy-phénylacétique par réduction de l'acide p-hydroxy-mandélique, procédé caractérisé en ce qu'on effectue la réduction au moyen d'HI en présence de phosphore rouge.

2. Procédé selon la revendication 1, caractérisé en ce que l'acide p-hydroxy-mandélique utilisé comme corps de départ pour la réduction est préparé in situ à partir du phénol et de l'acide glyoxylique.

3. Procédé selon l'une des revendications 1 et 2, caractérisé en ce que l'iodure d'hydrogène HI est mis en jeu sous la forme d'une solution aqueuse, de préférence sous la forme de l'acide iodhydrique aqueux à environ 57 % en poids qui bout à température constante.

4. Procédé selon la revendication 2, caractérisé en ce que l'acide glyoxylique est mis en jeu sous la forme de la solution aqueuse commerciale à environ 50 % en poids.